# DEMANDE DE BREVET EUROPEEN

(11) **EP 2 845 855 A1**
(43) Date de publication de la demande: **11.03.2015**
(21) Numéro de dépôt: 14188601.0
(22) Date de dépôt: 03.08.2009
(51) Int. Cl.: C07D 471/18, A61K 31/437, A61P 31/04

(54) **Nouveaux composés hétérocycliques azotés, leur préparation et leur utilisation comme médicaments antibactériens**

(30) Priorité: 03.10.2008 FR 0805472
(62) Demande divisionnaire de: 09786093.6
(71) Demandeur: ASTRA ZENECA HOLDING FRANCE, 92500 Rueil Malmaison (FR)
(72) Inventeur: Lampilas, Maxime, 75015 PARIS (FR); Rowlands, David, 78300 POISSY (FR); Ledoussal, Benoît, 22450 POMMERIT JAUDY (FR); Gourdel, Marie-Edith, 77176 SAVIGNY LE TEMPLE (FR); Renaud, Emilie, 93000 BOBIGNY (FR); Pierres, Camille, 75013 PARIS (FR); Kebsi, Adel, 94320 THIAIS (FR)
(74) Mandataire: Hirsch & Associés

(57) **Abrégé**

L'invention concerne des composés hétérocycliques azotés de formule générale (I) 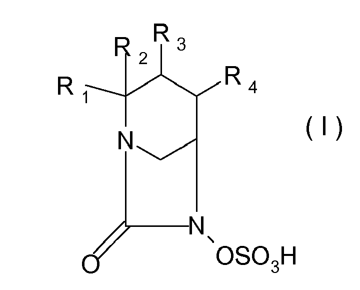 dans laquelle:
R₁ représente un radical (CH₂)ₙ-NH₂, n étant égal à 1 ou 2;
R₂ représente un atome d'hydrogène;
R₃ et R₄ forment ensemble un hétérocycle azoté à caractère aromatique à 5 sommets renfermant 1, 2 ou 3 atomes d'azote, substitué sur cet atome d'azote ou sur l'un de ces atomes d'azote par un groupe (CH₂)ₘ-(C(O))ₚ-R₅, m étant égal à 0, 1, 2 ou 3, p étant égal à 0 ou 1 et R₅ représentant un groupe hydroxy, auquel cas p est égal à 1, ou un groupe amino, alkyl(C₁-C₆) ou dialkyl(C₁-C₆) amino, ou un hétérocycle azoté à caractère aromatique à 5 ou 6 sommets renfermant 1 ou 2 atomes d'azote et, le cas échéant,un atome d'oxygène ou de soufre ; étant entendu que lorsque le sous-groupe (C(O))ₚ-R₅ forme un groupe carboxy, amino, alkyl(C₁-C₆) ou dialkyl(C₁-C₆) amino, m est différent de 0 ou 1;

sous forme libre et sous forme de zwitterions et de sels avec les bases et les acides minéraux ou organiques pharmaceutiquement acceptables,
leur préparation et leur utilisation comme médicaments antibactériens.

## Description

L'invention concerne des composés hétérocycliques azotés, leur préparation et leur utilisation comme médicaments antibactériens.

La demande WO 04/052891 décrit notamment des composés répondant à la formule suivante: 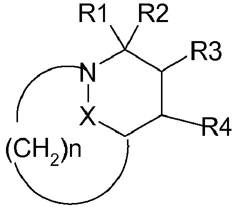 dans laquelle:
R₁ représente un atome d'hydrogène, un radical COOH, COOR, CN, (CH₂)_{n'}R₅, CONR₆R₇ ou 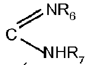
R est choisi dans le groupe constitué par un radical alkyle renfermant de 1 à 6 atomes de carbone, éventuellement substitué par un ou plusieurs atomes d'halogène ou par un radical pyridyle, un radical -CH₂-alkényle renfermant au total de 3 à 9 atomes de carbone, un groupe (poly)alkoxyalkyle renfermant 1 à 4 atomes d'oxygène et 3 à 10 atomes de carbone, un radical aryle renfermant de 6 à 10 atomes de carbone ou aralkyle renfermant de 7 à 11 atomes de carbone, le noyau du radical aryle ou aralkyle étant éventuellement substitué par un radical OH, NH₂, NO₂, alkyle renfermant de 1 à 6 atomes de carbone, alkoxy renfermant de 1 à 6 atomes de carbone ou par un ou plusieurs atomes d'halogène,
R₅ est choisi dans le groupe constitué par un radical COOH, CN, OH, NH₂, CO-NR₆R₇, COOR, OR, R étant défini comme ci-dessus,
R₆ et R₇ sont individuellement choisis dans le groupe constitué par un atome d'hydrogène, un radical alkyle renfermant de 1 à 6 atomes de carbone, alkoxy renfermant de 1 à 6 atomes de carbone, aryle renfermant de 6 à 10 atomes de carbone et aralkyle renfermant de 7 à 11 atomes de carbone et un radical alkyle renfermant de 1 à 6 atomes de carbone substitué par un radical pyridyle,
n' est égal à 1 ou 2,
R₃ et R₄ forment ensemble un phényl ou un hétérocycle à caractère aromatique à 5 ou 6 sommets renfermant de 1 à 4 héréroatomes choisis parmi l'azote, l'oxygène et le soufre, substitué par un ou plusieurs groupements R', R' étant choisi dans le groupe constitué par les radicaux -(O)ₐ-(CH₂)_{b}-(O)ₐ-CONR₆R₇, -(O)ₐ-(CH₂)_{b}-OSO₃H, -(O)ₐ-(CH₂)_{b}-SO₃H, - (O)ₐ-SO₂R, -(O)ₐ-SO₂-CHal₃, -(O)ₐ-(CH₂)_{b}-NR₆R₇, - (O)ₐ-(CH₂)_{b}-NH-COOR, -(CH₂)_{b}-COOH, -(CH₂)_{b}-COOR, -OR", OH, -(CH₂)_{b}- phényle et -(CH₂)_{b} - hétérocycle à caractère aromatique à 5 ou 6 sommets renfermant de 1 à 4 hétéroatomes choisis parmi l'azote, l'oxygène et le soufre, le phényle et l'hétérocycle étant éventuellement substitués par un ou plusieurs halogènes, alkyle renfermant de 1 à 6 atomes de carbone, alkoxy renfermant de 1 à 6 atomes de carbone ou CF₃, R, R₆ et R₇ étant tels que définis précédemment, R" étant choisi dans le groupe constitué par les radicaux alkyles renfermant de 1 à 6 atomes de carbone substitués par un ou plusieurs radicaux hydroxy, hydroxy protégés, oxo, halogène ou cyano, a étant égal à O ou 1 et b étant un entier de 0 à 6, étant entendu que lorsque R' est OH, R₁ représente le radical CONR₆R₇ dans lequel R₆ ou R₇ est un alkoxy renfermant de 1 à 6 atomes de carbone,
R₂ est choisi dans le groupe constitué par un atome d'hydrogène, un atome d'halogène et les radicaux R, S(O)ₘR, OR, NHCOR, NHCOOR et NHSO₂R, R étant tel que défini précédemment et m étant égal à 0,1 ou 2,
X représente un groupement divalent -C(O)-B- relié à l'atome d'azote par l'atome de carbone,
B représente un groupement divalent -O-(CH₂)_{n"}- lié au carbonyle par l'atome d'oxygène, un groupement -NR₈-(CH₂)_{n"}- ou -NR₈-O-relié au carbonyle par l'atome d'azote, n" est égal à 0 ou 1 et R₈ est choisi dans le groupe constitué par un atome d'hydrogène, un radical OH, R, OR, Y, OY, Y₁, OY₁, Y₂, OY₂, Y₃, O-CH₂-CH₂-S(O)m-R, SiRaRbRc et OSiRaRbRc, Ra, Rb et Rc représentant individuellement un radical alkyle linéaire ou ramifié renfermant de 1 à 6 atomes de carbone ou un radical aryle renfermant de 6 à 10 atomes de carbone, et R et m étant définis comme précédemment,
Y est choisi dans le groupe constitué par les radicaux COH, COR, COOR, CONH₂, CONHR, CONHOH, CONHSO₂R, CH₂COOH, CH₂COOR, CHF-COOH, CHF-COOR, CF2-COOH, CF2-COOR, CN, CH₂CN, CH₂CONHOH, CH₂CONHCN, CH₂tétrazole, CH₂tétrazole protégé, CH₂SO₃H, CH₂SO₂R, CH₂PO(OR)₂, CH₂PO(OR)(OH), CH₂PO(R)(OH) et CH₂PO(OH)₂,
Y₁ est choisi dans le groupe constitué par les radicaux SO₂R, SO₂NHCOH, SO₂NHCOR, SO₂NHCOOR, SO₂NHCONHR, SO₂NHCONH₂ et SO₃H,
Y₂ est choisi dans le groupe constitué par les radicaux PO(OH)₂, PO(OR)₂, PO(OH)(OR) et PO(OH)(R),
Y₃ est choisi dans le groupe constitué par les radicaux tétrazole, tétrazole substitué par le radical R, squarate, NH ou NR tétrazole, NH ou NR tétrazole substitué par le radical R, NHSO₂R et NRSO₂R, CH₂ tétrazole et CH₂ tétrazole substitué par R, R étant défini comme ci-dessus, et
n est égal à 1 ou 2,
ainsi que les sels de ces composés avec des bases ou des acides minéraux ou organiques.

Les atomes de carbone asymétriques contenus dans les composés de formule (I) peuvent indépendamment les uns des autres présenter la configuration R, S ou RS et les composés de formule (I) se présentent donc sous la forme d'énantiomères purs ou de diastéréoisomères purs ou sous la forme d'un mélange d'énantiomères notamment de racémates, ou de mélanges de diastéréoisomères.

En outre, le substituant R₁, R₂, ou R₄ pris individuellement d'une part et X d'autre part pouvant être en position cis et/ou trans par rapport au cycle sur lequel ils sont fixés, les composés de formule (I) se présentent sous la forme d'isomères cis ou d'isomères trans ou de mélanges.

Par ailleurs, la demande WO 02/100860 décrit des composés apparentés.
La demanderesse a découvert que parmi les composés décrits dans la demande WO 04/052891, certains composés particuliers, dont aucun n'est décrit dans la partie expérimentale de cette demande, possèdent des propriétés antibactériennes tout à fait inattendues.

Le caractère unique des composés de l'invention réside dans le fait qu'ils présentent une excellente activité sur *Pseudomonas aeruginosa,* une souche bactérienne fréquemment rencontrée dans les infections nocosomiales ainsi que chez les patients souffrant de mucoviscidose.

Cette activité intéressante et inattendue n'est pas présente dans les composés les plus proches préparés dans la demande WO 04/052891. Elle est illustrée plus loin dans la partie expérimentale.

Par ailleurs, les composés de l'invention se sont montrés actifs sur des modèles d'infection animale, y compris sur des souches habituellement résistantes aux antibiotiques communément utilisés. Les composés de l'invention sont capables de contrecarrer les principaux mécanismes de résistance des bactéries que sont les β-lactamases, les pompes à efflux et les mutations des porines.

Les composés de l'invention sont des composés répondant à la formule ci-dessus, dans laquelle R₂ représente un atome d'hydrogène, X représente un groupement divalent C(O)NR₈ dans lequel R₈ est un radical OY₁, Y₁ étant un radical SO₃H, et surtout comportant la combinaison particulière de substituants R₁, R₃, R₄ suivante:
R₁ représente un radical alkyle substitué par un radical amino et R₃ et R₄ forment ensemble un hétérocycle azoté à caractère aromatique à 5 sommets substitué par un groupe comportant ou consistant en un substituant polaire de type amino ou hétérocycle aromatique aminé ou carboxy.

L'invention a ainsi pour objet les composés de formule générale (I), sous leurs formes énantiomères ou diastéroisomères ou isomères cis ou trans possibles, ou de mélanges: 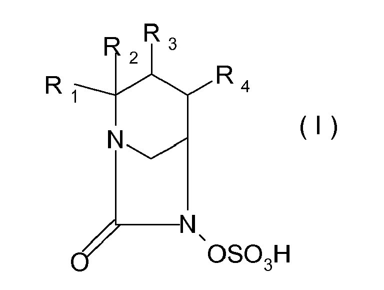 dans laquelle:
R₁ représente un radical (CH₂)ₙ-NH₂, n étant égal à 1 ou 2;
R₂ représente un atome d'hydrogène;
R₃ et R₄ forment ensemble un hétérocycle azoté à caractère aromatique à 5 sommets renfermant 1, 2 ou 3 atomes d'azote, substitué sur cet atome d'azote ou sur l'un de ces atomes d'azote par un groupe (CH₂)ₘ-(C(O))ₚ-R₅, m étant égal à 0, 1, 2 ou 3, p étant égal à 0 ou 1 et R₅ représentant un groupe hydroxy, auquel cas p est égal à 1, ou un groupe amino, alkyl(C₁-C₆) ou dialkyl(C₁-C₆) amino, ou un hétérocycle azoté à caractère aromatique à 5 ou 6 sommets renfermant 1 ou 2 atomes d'azote et, le cas échéant,un atome d'oxygène ou de soufre ;
étant entendu que lorsque le sous-groupe (C(O))ₚ-R₅ forme un groupe carboxy, amino ou alkyl(C₁-C₆) ou dialkyl(C₁-C₆) amino, m est différent de 0 ou 1;
sous forme libre et sous forme de zwitterions et de sels avec les bases et les acides minéraux ou organiques pharmaceutiquement acceptables.

Par radical alkyle renfermant de 1 à 6 atomes de carbone, on entend notamment le radical méthyle, éthyle, propyle, isopropyle, ainsi que butyle, pentyle ou hexyle linéaire ou ramifié.

Par hétérocycle à caractère aromatique à 5 sommets renfermant 1, 2 ou 3 atomes d'azote, on entend ceux choisis dans la liste qui suit, les deux liaisons symbolisant la jonction avec le cycle azoté formé par R₃ et R₄: 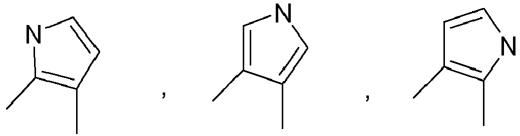 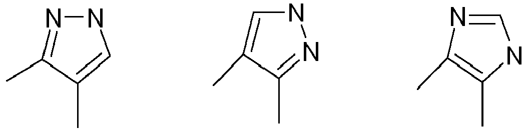 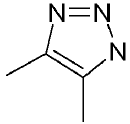 Par hétérocycle azoté à caractère aromatique à 5 ou 6 sommets renfermant 1 ou 2 atomes d'azote et, le cas échéant, 1 atome d'oxygène ou de soufre, on entend ceux du type représenté ci-dessus, ou un cycle oxazole ou thiazole, ou un cycle à 6 sommets de type pyridine, pyrazine, pyrimidine ou pyridazine, l'hétérocycle étant fixé à la chaîne ou à l'hétérocycle formé par R₃ et R₄ par un atome d'azote ou par un atome de carbone. Parmi les sels d'acides des produits de formule (I), on peut citer entre autres, ceux formés avec les acides minéraux tels que les acides chlorhydrique, bromhydrique, iodhydrique, sulfurique ou phosphorique ou avec les acides organiques comme l'acide formique, acétique, trifluoroacétique, propionique, benzoïque, maléique, fumarique, succinique, tartrique, citrique, oxalique, glyoxylique, aspartique, alcanesulfoniques, tels que les acides méthane et éthane sulfoniques, arylsulfoniques tels que les acides benzène et paratoluènesulfoniques.

Parmi les sels de bases des produits de formule (I), on peut citer, entre autres, ceux formés avec les bases minérales telles que, par exemple, l'hydroxyde de sodium, de potassium, de lithium, de calcium, de magnésium ou d'ammonium ou avec les bases organiques telles que, par exemple, la méthylamine, la propylamine, la triméthylamine, la diéthylamine, la triéthylamine, la N,N-diméthyléthanolamine, le tris (hydroxyméthyl)aminométhane, l'éthanolamine, la pyridine, la picoline, la dicyclohexylamine, la morpholine, la benzylamine, la procaïne, la lysine, l'arginine, l'histidine, la N-méthylglucamine, ou encore les sels de phosphonium, tels que les alkylphosphonium, les arylphosphoniums, les alkylarylphosphonium, les alkénylarylphosphonium ou les sels d'ammoniums quaternaires tels que le sel de tétra n-butylammonium.

Les atomes de carbone asymétriques contenus dans les composés de formule (I) peuvent indépendamment les uns des autres présenter la configuration R, S ou RS et les composés de formule (I) se présentent donc sous la forme d'énantiomères purs ou de diastéréoisomères purs ou sous la forme d'un mélange d'énantiomères notamment de racémates, ou de mélanges de diastéréoisomères.

En outre, le substituant R₁ d'une part et la chaîne -C(O)-N(OSO₃H)- d'autre part pouvant être en position cis et/ou trans par rapport au cycle sur lequel ils sont fixés, les composés de formule (I) se présentent sous la forme d'isomères cis ou d'isomères trans ou de mélanges.

Parmi les composés de formule (I), l'invention a notamment pour objet les composés dans lesquels R₃ et R₄ forment ensemble un hétérocycle pyrazolyle substitué.

Parmi les composés de formule (I), l'invention a notamment pour objet ceux dans lesquels R₁ est un groupement (CH₂)ₙ-NH₂, n étant égal à 1 et l'hétérocycle formé par R₃ et R₄ est substitué par un groupement (CH₂)ₘ-(C(O))ₚ-R₅ tel que défini précédemment, et plus particulièrement parmi ceux-ci, ceux dans lesquels R₅ représente un groupe amino, alkyl(C₁-C₆) ou dialkyl (C₁-C₆) amino, m et p étant tels que définis précédemment.

Parmi les composés de formule (I), l'invention a tout particulièrement pour objet les composés décrits plus loin

Parmi les composés de formule (I), l'invention a tout particulièrement pour objet les composés décrits plus loin dans la partie expérimentale et notamment ceux dont les noms suivent:
- la trans 8-(aminométhyl)-2-carbamoyle-4,8-dihydro-5-(sulfooxy)-4,7-méthano-7H-pyrazolo[3,4-e] [1,3] diazépin-6(5H)-one,
- la trans 8-(aminométhyl)-2-diméthylcarbamoyle-4,8-dihydro-5-(sulfooxy)-4,7-méthano-7H-pyrazolo[3,4-e] [1,3] diazépin-6(5H)-one,
- la trans 8-(aminométhyl)-2-méthylcarbamoyle-4,8-dihydro-5-(sulfooxy)-4,7-méthano-7H-pyrazolo[3,4-e] [1,3] diazépin-6(5H)-one,
- la trans 8-(aminométhyl)-1-(2-aminoéthyl)-4,8-dihydro-5-(sulfooxy)-4,7-méthano-7H-pyrazolo[3,4-e] [1,3] diazépin-6(5H)-one,
- la trans 8-(aminométhyl)-2-(2-aminoéthyl)-4,8-dihydro-5-(sulfooxy)-4,7-méthano-7H-pyrazolo[3,4-e] [1,3] diazépin-6(5H)-one,
- la trans 8-(aminométhyl)-2-(2-pyridinyl)-4,8-dihydro-5-(sulfooxy)-4,7-méthano-7H-pyrazolo[3,4-e] [1,3] diazépin-6(5H)-one,
- le trans 8-(aminométhyl)-5,6-dihydro-6-oxo-5-(sulfooxy)-4H-4,7-méthanopyrazolo[3,4-e] [1,3] diazépin-2(8H)-acétamide,
sous forme libre, de zwitterions et de sels avec les bases et les acides minéraux ou organiques pharmarceutiquement acceptables, et sous leurs formes énantiomères ou diastéroisomères ou isomères cis ou trans possibles, ou de mélanges.

Un autre objet de l'invention est un procédé de préparation des composés de formule (I), caractérisé en ce que l'on traite un composé de formule (II) : 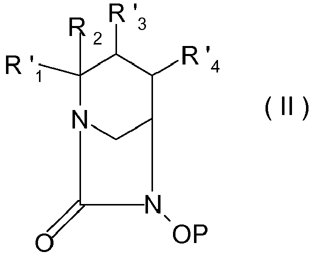 dans laquelle R'₁ représente un radical R₁ dans lequel l'atome d'azote est protégé, R₂ est tel que défini plus haut, R'₃ et R'₄ forment ensemble un hétérocycle azoté à caractère aromatique à 5 sommets renfermant 1, 2 ou 3 atomes d'azote et P représente un groupement protecteur du radical hydroxy, en présence d'une base, par un composé de formule (III) :

X-(CH₂)ₘ-(C(O))ₚ-R'₅ (III)

dans laquelle X représente un atome d'halogène ou un groupement OH pouvant être activé, m et p sont tels que définis plus haut et R'₅ représente un radical R₅ dans lequel le groupe réactif amino ou carboxy est le cas échéant protégé, pour obtenir un composé de formule (IV) : 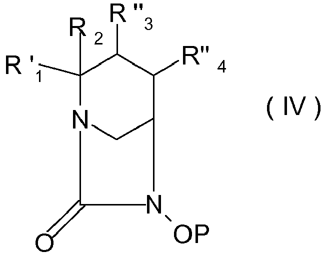 dans laquelle R'₁, R₂ et P sont tels que définis plus haut et R"₃ et R"₄ forment ensemble un hétérocycle azoté à caractère aromatique à 5 sommets tel que défini plus haut pour R₃ et R₄, substitué par un groupement (CH₂)ₘ-(C(O))ₚ-R'₅, m, p et R'₅ étant tels que définis plus haut,
puis déprotège le radical hydroxy et soumet le composé obtenu à une réaction de sulfatation par action de SO₃ complexé,
puis, le cas échéant, soumet le composé obtenu à une ou plusieurs des réactions suivantes, dans un ordre approprié :
- déprotection de la ou des fonctions aminées et le cas échéant carboxy présentes,
- salification,
- échange d'ions,
- dédoublement ou séparation de diastéréoisomères.

La protection préalable de l'amine au niveau de R'₁ et R'₅ est notamment effectuée sous forme de dérivés benzylés ou tritylés, sous forme de carbamates, notamment d'allyle, benzyle, phényle ou tertbutyle, ou encore sous forme de dérivés silylés tels que les dérivés tertbutyle diméthyl, triméthyl, triphényl ou encore diphényltertbutyl-silyle, ou encore de dérivés phénylsulfonylalkyle ou cyanoalkyle.

La déprotection peut être effectuée par différentes méthodes connues de l'homme du métier, selon la nature du groupement protecteur. Elle peut notamment être effectuée par action d'un acide, par exemple l'acide trifluoroacétique, le composé déprotégé étant alors obtenu sous forme de sel de l'acide. Elle peut encore être effectuée par hydrogénolyse ou à l'aide de complexes solubles du Palladium O ou par action du fluorure de tétrabutylammonium ou par réduction.Une illustration est fournie plus loin dans la partie expérimentale.

La protection préalable du carboxy au niveau de R'₅ est notamment effectuée sous forme de dérivés de type esters, notamment d'alkyle, allyle, benzyle, benzhydryle ou p-nitro benzyle.

La déprotection peut être effectuée par différentes méthodes connues de l'homme du métier, par exemple par saponification, hydrolyse acide, hydrogénolyse ou clivage à l'aide de complexes solubles du Palladium 0.

La base en présence de laquelle on fait réagir les composés de formules (II) et (III) peut par exemple être un carbonate alcalin mais d'autres bases connues de l'homme du métier peuvent être utilisées.

La protection préalable de l'hydroxy du composé de formule (II) est effectuée de manière classique, sous forme d'éthers, d'esters ou de carbonates. Les éthers peuvent être des éthers d'alkyle ou d'alkoxyalkyle, de préférence des éthers de méthyle ou de méthoxyéthoxyméthyle, des éthers d'aryle ou de préférence d'aralkyle, par exemple de benzyle, ou des éthers silylés, par exemple les dérivés silylés cités plus haut. Les esters peuvent être n'importe quel ester clivable connu de l'homme du métier et de préférence l'acétate, le propionate ou le benzoate ou p-nitrobenzoate. Les carbonates peuvent être par exemple des carbonates de méthyle, tertbutyle, allyle, benzyle ou p-nitrobenzyle.

La déprotection est effectuée par les moyens connus de l'homme du métier, notamment la saponification, l'hydrogénolyse, le clivage par des complexes solubles du Palladium O, l'hydrolyse en milieu acide ou encore, pour les dérivés silylés, le traitement par le fluorure de tétrabutylammmonium. Une illustration est fournie plus loin dans la partie expérimentale.

L'activation éventuelle de l'hydroxy du composé de formule (III) est réalisée sous forme de mésylate ou tosylate, dans des conditions connues de l'homme du métier.

La réaction de sulfatation est effectuée par action des complexes de SO₃ tels que SO₃-pyridine ou SO₃-diméthylformamide, en opérant dans la pyridine ou dans le diméthylformamide, le sel formé, par exemple le sel de pyridine, pouvant ensuite être échangé par exemple par un sel d'une autre amine, d'un ammonium quaternaire ou d'un métal alcalin. Une illustration est fournie dans la partie expérimentale.

La salification par les acides est le cas échéant réalisée par addition d'un acide en phase soluble au composé. La salification par les bases de la fonction sulfooxy peut être réalisée à partir du sel d'amine et notamment de pyridine obtenu lors de l'action du complexe SO₃-amine et on obtient les autres sels à partir de ce sel d'amine. On peut notamment opérer par échange d'ions sur résine.

La séparation des énantiomères et diastéréoisomères peut être réalisée selon les techniques connues de l'homme du métier, notamment la chromatographie, sur phase chirale ou non.

Des exemples de conditions utilisables sont aussi décrits dans la demande WO 04/052891 ou encore dans la demande WO 02/100860.

Les composés de formule (I) dans lesquels m est égal à 0, p est égal à 1 et R₅ représente R"₅, R"₅ représentant un groupe amino, alkyl(C₁-C₆) ou dialkyl(C₁-C₆) amino, peuvent encore être obtenus par un procédé caractérisé en ce que l'on traite un composé de formule (II) telle que définie plus haut, en présence d'une base, par le diphosgène puis par une amine de formule

H-R"₅

dans laquelle R"₅ a les valeurs de R₅ ci-dessus, pour obtenir un composé de formule (IV') : 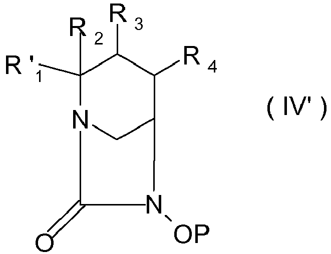 dans laquelle R'₁, R₂ et P sont tels que définis plus haut et R₃ et R₄ forment ensemble un hétérocycle azoté à caractère aromatique à 5 sommets tel que défini plus haut substitué par un groupement -C(O)-R"₅, R"₅ étant tel que défini plus haut,puis poursuit la synthèse comme décrit plus haut dans le cas du composé de formule (IV).

La base utilisée lors de l'action du diphosgène peut notamment être une amine tertiaire telle que la triéthylamine

Ces mêmes composés de formule (I) peuvent encore, le cas échéant, être obtenus par un procédé caractérisé en ce que l'on traite un composé de formule (II) telle que définie plus haut, par le triméthylsilyl isocyanate ou par un isocyanate de formule

alkyl(C₁-C₆)-N=C=O

pour obtenir un composé correspondant de formule (IV), puis poursuit la synthèse comme décrit plus haut.

Les composés de formule (I) dans lesquels R₅ représente un hétérocycle peuvent être obtenus par les différentes réactions connues de l'homme du métier pour former les liaisons C-N et notamment par catalyse au palladium ou au cuivre comme celle décrite dans l'un des exemples ci-après

Comme indiqué plus haut, les composés de formule générale (I) possèdent une excellente activité antibiotique sur *Pseudomonas aeruginosa* ainsi que sur des modèles d'infection animale par des souches résistantes aux agents antibactériens communément utilisés. Cette activité antibiotique remarquable et inattendue n'avait pas été observée pour les composés décrits dans la demande WO 04/052891 et notamment pour les composés structurellement proches. Ceci est illustré plus loin.
Ces propriétés rendent aptes lesdits composés, sous forme libre et de zwitterions ou de sels d'acides et de bases pharmaceutiquement acceptables, à être utilisés comme médicaments dans le traitement des infections sévères à *Pseudomonas,* notamment les infections nosocomiales et, d'une manière générale, les infections majeures chez les sujets à risques. Il peut en particulier s'agir d'infections des voies respiratoires, par exemple la pneumonie aiguë ou les infections chroniques des voies inférieures, les infections du sang, par exemple les septicémies, les infections aiguës ou chroniques des voies urinaires, celles du système auditif, par exemple l'otite externe maligne, ou l'otite chronique suppurante, celles de la peau et des tissus mous, par exemple les dermatites, les plaies infectées, la folliculite, la pyodermite, les formes rebelles d'acnée, les infections des yeux, par exemple l'ulcère de la cornée, celles du système nerveux, notamment les méningites et les abcès du cerveau, les infections cardiaques telles que l'endocardite, les infections des os et des articulations telles que la pyoarthrose sténoarticulaire, l'ostéomyélite vertébrale, la symphysite pubienne, les infections du tube gastro-intestinal, telles que l'entérocolite nécrosante et les infections péri-rectales.

La présente invention a donc également pour objet, à titre de médicaments et notamment de médicaments antibiotiques, les composés de formule (I) tels que définis ci-dessus, sous forme libre et sous forme de zwitterions et de sels avec les bases et les acides minéraux ou organiques pharmaceutiquement acceptables.

Parmi les composés de formule (I), l'invention a notamment pour objet, à titre de médicaments, les composés dans lesquels R₃ et R₄ forment ensemble un hétérocycle pyrazolyle substitué.

Parmi les composés de formule (I), l'invention a plus particulièrement pour objet, à titre de médicaments, les composés dans lesquels R₁ est un groupement (CH₂)ₙ-NH₂, n étant égal à 1 et l'hétérocycle formé par R₃ et R₄ est substitué par un groupement (CH₂)ₘ-(C(O))ₚ-R₅ tel que défini précédemment, et plus particulièrement parmi ceux-ci, ceux dans lesquels R₅ représente un groupe amino, alkyl(C₁-C₆) ou dialkyl(C₁-C₆) amino, m et p étant tels que définis précédemment.

Parmi les composés de formule (I), l'invention a tout particulièrement pour objet, à titre de médicament, les composés dont les noms suivent:
- la trans 8-(aminométhyl)-2-carbamoyle-4,8-dihydro-5-(sulfooxy)-4,7-méthano-7H-pyrazolo[3,4-e] [1,3] diazépin-6(5H)-one,
- la trans 8-(aminométhyl)-2-diméthylcarbamoyle-4,8-dihydro-5-(sulfooxy)-4,7-méthano-7H-pyrazolo[3,4-e] [1,3] diazépin-6(5H)-one,
- la trans 8-(aminométhyl)-2-méthylcarbamoyle-4,8-dihydro-5-(sulfooxy)-4,7-méthano-7H-pyrazolo[3,4-e] [1,3] diazépin-6(5H)-one,
- la trans 8-(aminométhyl)-1-(2-aminoéthyl)-4,8-dihydro-5-(sulfooxy)-4,7-méthano-7H-pyrazolo[3,4-e] [1,3] diazépin-6(5H)-one,
- la trans 8-(aminométhyl)-2-(2-aminoéthyl)-4,8-dihydro-5-(sulfooxy)-4,7-méthano-7H-pyrazolo[3,4-e] [1,3] diazépin-6(5H)-one,
- la trans 8-(aminométhyl)-2-(2-pyridinyl)-4,8-dihydro-5-(sulfooxy)-4,7-méthano-7H-pyrazolo[3,4-e] [1,3] diazépin-6(5H)-one,
- le trans 8-(aminométhyl)-5,6-dihydro-6-oxo-5-(sulfooxy)-4H-4,7-méthanopyrazolo[3,4-e] [1,3] diazépin-2(8H)-acétamide,
sous forme libre, de zwitterions et de sels avec les bases et les acides minéraux ou organiques pharmarceutiquement acceptables, et sous leurs formes énantiomères ou diastéroisomères ou isomères cis ou trans possibles, ou de mélanges.

L'invention a aussi pour objet les compositions pharmaceutiques renfermant comme principe actif, au moins un des composés selon l'invention tels que définis ci-dessus. Ces compositions peuvent être administrées par voie buccale, rectale, parentérale, notamment intramusculaire, ou par voie locale en application topique sur la peau et les muqueuses.

Les compositions selon l'invention peuvent être solides ou liquides et se présenter sous les formes pharmaceutiques couramment utilisées en médecine humaine comme par exemple, les comprimés simples ou dragéifiés, les gélules, les granulés, les suppositoires, les préparations injectables, les pommades, les crèmes, les gels; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Ces compositions peuvent notamment se présenter sous forme d'un lyophilisat destiné à être dissout extemporanément dans un véhicule approprié, par exemple, de l'eau stérile apyrogène.

La dose administrée est variable selon l'affection traitée, le sujet en cause, la voie d'administration et le produit considéré. Elle peut être, par exemple, comprise entre 0,250 g et 10 g par jour, par voie orale chez l'homme, avec le produit décrit à l'exemple 1, 4 ou 5 ou encore comprise entre 0,25 g et 10 g par jour par voie intramusculaire ou intraveineuse.

Les produits de formule (I) peuvent également être utilisés comme désinfectants des instruments chirurgicaux.

Les exemples suivants illustrent l'invention.

### Exemple 1 : Sel de sodium et de trifluoroacétate de la trans 8-(aminométhyl)-2-carbamoyle-4,8-dihydro-5-(sulfooxy)-4,7-méthano-7H-pyrazolo[3,4-e] [1,3] diazépin-6(5H)-one

### Stade A

### Trans-8-(hydroxyméthyl)-4,8-dihydro-6-oxo-5-(phénylméthoxy)-4,7-méthano-7H-pyrazolo[3,4-e][1,3]diazépin-6(5H)-one

L'ester trans-4,5,6,8-tétrahydro-6-oxo-5-(phénylméthoxy)-4,7-méthano-7H-pyrazolo[3,4-e][1,3]diazépine-8-carboxylate de méthyle décrit dans la demande WO2004/052891 (Exemple 1, stade K) (5g, 15.2mmol) est mis en solution dans un mélange méthanol/tétrahydrofurane anhydres 1/1 (100mL), sous azote. Du NaBH₄ (2.3g, 60.9mmol) est alors ajouté par portions. Après une nuit d'agitation à température ambiante, le mélange réactionnel est traité avec une solution aqueuse 10% de NaH₂PO₄ (100mL). Après évaporation à sec, le mélange réactionnel est repris dans l'eau. Le précipité formé est agité une nuit dans la glace, puis filtré et séché sous pression réduite en présence de P₂O₅, pour donner le composé attendu (3.30g, 11.0mmol, 72%) sous forme de poudre blanche.

MS (ES(+)) : m/z [M+H]⁺ = 301

¹H RMN (400MHz, DMSO-d₆ : δ (ppm) = 3.18-3.50 (ABX, 2H, N-CH₂-CH-N), 3.65-3.76 (ABX, 2H, N-CH-CH₂-OH), 4.34 (t, 1H, N-CH-CH₂-OH), 4.46 (d, 1H, N-CH₂-CH-N), 4.88 (s, 2H, CH₂-Ph), 7.29-7.43 (m, 5H, Ph), 7.66 (s, 1H, H pyrazole), 12.72 (broad, 1H, OH).

### Stade B

### Trans [[4,5,6,8-tétrahydro-6-oxo-5-(phénylméthoxy)-4,7-méthano-7H-pyrazolo[3,4-e] [1,3] diazépin-8-yl]méthyl]-carbamate de 1,1-diméthyle

L'alcool obtenu au stade A de l'exemple 1 (1.73g, 5.76mmol) est mis en solution dans la pyridine anhydre (35mL) sous azote, à 0°C, puis du chlorure de méthanesulfonyle (1.78mL, 23mmol) est ajouté goutte à goutte. Après 2h30 d'agitation à température ambiante, le mélange réactionnel est traité avec une solution aqueuse saturée de chlorure d'ammonium (100mL), puis extrait à l'acétate d'éthyle. Les phases organiques combinées sont ensuite lavées avec une solution aqueuse saturée de chlorure d'ammonium, séchées puis concentrées sous pression réduite pour donner le dérivé dimésylé attendu sous forme d'huile jaune.

L'intermédiaire dimésylé est mis en solution dans du diméthylformamide anhydre (45mL), sous azote, en présence d'azoture de sodium (1.12g, 17.3mmol). Le mélange réactionnel est chauffé à 70°C pendant 24h. 1 équivalent. d'azoture est ajouté le cas échéant pour que la conversion soit complète. Lorsque la réaction est complète, le mélange est traité avec une solution aqueuse 10% de NaH₂PO₄ (100mL) puis extrait au dichlorométhane. Les phases organiques combinées sont séchées puis concentrées sous pression réduite pour donner l'azoture attendu sous forme d'huile jaune.

L'intermédiaire est mis en réaction, sous azote, dans l'éthanol absolu (17.5mL), puis sont ajoutés successivement du di-tert-butyl dicarbonate (1.38g, 6.34mmol), du triéthylsilane (1.38mL, 8.64mmol) et de l'hydroxyde de palladium sur charbon 10% (52mg). Après une nuit à température ambiante, le mélange réactionnel est filtré puis concentré pour donner une huile jaune brute. Ce brut est purifié par chromatographie sur colonne de silice (éluant gradient CH₂Cl₂/MeOH 100/0 à 95/5 par 1%) pour conduire au composé attendu (1.36g, 3.40mmol, 34%) sous forme de solide blanc.

MS (ES(+)) : m/z [M+H]⁺ = 401

¹H RMN (400MHz, MeOH-d₄) : δ (ppm) = 1.51 (s, 9H, C(CH₃)₃), 3.21-3.59 (m, 4H, N-CH₂-CH-N et N-CH-CH₂-NHBoc), 4.36 (m, 1H, N-CH-CH₂-OH), 4.46 (m, 1H, N-CH₂-CH-N), 4.99 (AB, 2H, CH₂-Ph), 7.41-7.52 (m, 5H, Ph), 7.63 (s, 1H, H pyrazole).

### Stade C

### Trans [[2-carbamoyle-4,5,6,8-tétrahydro-6-oxo-5-(phénylméthoxy)-4,7-méthano-7H-pyrazolo[3,4-e] [1,3] diazépin-8-yl]méthyl]-carbamate de 1,1-diméthyléthyle

Sous azote, l'amine obtenue au stade B de l'exemple 1 (100mg, 0.250mmol) est mise en solution dans le dichlorométhane (13mL). A 0°C, la triéthylamine (70µL, 0.500mmol) est ajoutée, suivie du diphosgène (45µL, 0.376mmol) ajouté par goutte à goutte rapide. Après 2h30 d'agitation à 0°C, l'ammoniaque (20% aqueux, 0.4mL) est ajoutée rapidement et le milieu est agité vigoureusement à température ambiante pendant 1h.

Le milieu est transvasé dans une ampoule à décanter, rincé par du dichlorométhane (5mL), puis lavé par une solution aqueuse de phosphate de sodium à 10% (10mL). La phase aqueuse est extraite par le dichlorométhane (10mL). Les phases organiques sont rassemblées, lavées par une solution saturée de NaCl, séchées puis concentrées sous pression réduite pour donner, après chromatographie sur colonne de silice (éluant CH₂Cl₂/acétate d'éthyle 70/30), le dérivé attendu (94mg, 0.212mmol, 85%) sous forme d'un solide beige.

MS (ES (+)) : m/z [M+H]⁺ = 443

¹H NMR (400 MHz, CDCl₃) : δ (ppm) = 1.44 (s, 9H, C(CH₃)3), 3.09 (dd, 1H, N-CH₂-CH-N), 3.32 (m, 2H, CH-CH₂-NHBoc), 3.72 (dd, 1H, N-CH₂-CH-N), 3.98 (d, 1H, N-CH₂-CH-N), 4.59 (m, 1H, CH-CH₂-NHBoc), 4.92 (AB, 2H, N-O-CH₂-Ph), 5.93 (broad, 1H, NH), 6.95 (broad, 1H, NH), 7.37-7.41 (m, 5H, Ph), 8.03 (s, 1H, H pyrazole).

### Stade D

### Sel de pyridinium du trans [[2-carbamoyle-4,5,6,8-tétrahydro-6-oxo-5-(sulfooxy)-4,7-méthano-7H-pyrazolo[3,4-e] [1,3] diazépin-8-yl]méthyl]-carbamate de 1,1-diméthyléthyle

Sous azote, le dérivé obtenu au stade C (94mg, 0.212mmol) est mis en solution dans le diméthylformamide (0.3mL) et le dichlorométhane (0.9mL), puis du palladium 10% sur charbon à 50% en eau (68mg, 0.032mmol) est ajouté. Après purge vide/azote, le mélange réactionnel est placé sous atmosphère d'hydrogène jusqu'à disparition du produit de départ en HPLC. Le mélange est alors concentré sous vide puis co-évaporé avec du dichlorométhane anhydre, enfin séché sous pression réduite en présence de P₂O₅ pendant 2h, pour donner l'intermédiaire débenzylé attendu.

Le dérivé débenzylé est repris dans de la pyridine anhydre (0.6mL) en présence du complexe pyridine / sulfure de trioxyde (68mg, 0.425mmol). Le mélange réactionnel est ensuite agité à température ambiante jusqu'à conversion complète en HPLC, puis évaporé à sec après traitement par ajout d'eau. Le brut réactionnel est chromatographié sur colonne de silice (éluant gradient CH₂Cl₂/MeOH 100/0 à 80/20 par 5%) pour donner le produit attendu (50mg, 0.093mmol, 43%) sous forme d'un solide blanc.

MS (ES (-)) : m/z [M⁻] = 431

¹H NMR (400MHz, MeOH-d₄) : δ (ppm) = 1.52 (s, 9H, C (CH₃)₃), 3.41-3.53, 3.62-3.75 (m, 4H, N-CH₂-CH-N et CH-CH₂-NHBoc), 4.64 (m, 1H, CH-CH₂-NHBoc), 4.98 (d, 1H, N-CH₂-CH-N), 8.00 (m, 2H, Py), 8.28 (s, 1H, H pyrazole), 8.74 (m, 1H, Py), 8.95 (m, 2H, Py)

### Stade E

### Sel de sodium du trans [[2-carbamoyle-4,5,6,8-tétrahydro-6-oxo-5-(sulfooxy)-4,7-méthano-7H-pyrazolo[3,4-e] [1,3] diazépin-8-yl]méthyl]-carbamate de 1,1-diméthyléthyle

Une suspension de 6g de résine DOWEX 50WX8 dans une solution de soude 2N (30mL) est agitée pendant 1h, puis versée sur une colonne à chromatographie. La colonne est conditionnée à l'eau déminéralisée jusqu'à pH neutre, puis avec un mélange eau/THF 90/10. Le dérivé obtenu au stade D de l'exemple 1 (49mg, 0.091mmol) est dissout dans un minimum de méthanol, déposé sur la colonne, puis élué avec un mélange eau/THF 90/10. Les fractions contenant le substrat sont réunies, congelées et lyophilisées pour conduire au sel de sodium attendu (44mg, 0.091mmol, 100%) sous forme d'un solide beige.

MS (ES (-)) : m/z [M-H]⁻ = 431

¹H NMR (400MHz, MeOH-d₄) : δ (ppm) = 1.52 (s, 9H, C(CH₃)₃), 3.41-3.53, 3.62-3.75 (m, 4H, N-CH₂-CH-N et CH-CH₂-NHBoc), 4.64 (m, 1H, CH-CH₂-NHBoc), 4.98 (d, 1H, N-CH₂-CH-N), 8.29 (s, 1H, H pyrazole).

### Stade F

### Sel de sodium et de trifluoroacétate de la trans 8-(aminométhyl)-2-carbamoyle-4,8-dihydro-5-(sulfooxy)-4,7-méthano-7H-pyrazolo[3,4-e] [1,3] diazépin-6(5H)-one

Une solution d'acide trifluoroacétique (2.4mL) dans le dichlorométhane (2.4mL) est ajoutée goutte à goutte à une solution du sel de sodium obtenu au stade E (42mg, 0.092mmol) dans le dichlorométhane (1.2mL) sous azote et refroidie à 0°C. La réaction est maintenue sous agitation pendant 1h à température ambiante. Le mélange est évaporé à sec et repris dans l'eau pour donner un précipité beige. Le précipité est filtré, puis lavé par l'éthanol pour donner le dérivé attendu (12mg, 0.026mmol, 28%) sous forme d'un solide beige.

¹H NMR (400 MHz, DMSO-d₆) : δ (ppm) = 3.18 (m, 1H, N-CH₂-CH-N), 3.40-3.47 (m, 3H, N-CH₂-CH-N et CH-CH₂-NH₃⁺), 4.68 (m, 1H, CH-CH₂-NH₃⁺), 4.85 (d, 1H, N-CH₂-CH-N), 7.79 (broad, 1H, CONH₂), 7.87 (broad, 1H, CONH₂), 8.09 (broad, 3H, NH₃⁺), 8.26 (s, 1H, H pyrazole)

### Exemple 2 : Sel de sodium et de trifluoroacétate de la trans 8-(aminométhyl)-2-diméthylcarbamoyle-4,8-dihydro-5-(sulfooxy)-4,7-méthano-7H-pyrazolo[3,4-e] [1,3] diazépin-6(5H)-one

### Stade A

### Trans [[4,5,6,8-dihydro-2-diméthylcarbamoyle-6-oxo-5-(phényl méthoxy)-4,7-méthano-7H-pyrazolo[3,4-e] [1,3] diazépin-8-yl]méthyl]-carbamate de 1,1-diméthyléthyle

En procédant comme indiqué au stade C de l'exemple 1, l'utilisation du dérivé obtenu au stade B de l'exemple 1 (200mg, 0.501mmol), de dichlorométhane (26mL), de triéthylamine (140µL, 1.00mmol), de diphosgène (91µL, 0.751mmol) et de diméthylamine (40wt.% aqueux, 0.634mL, 5.01mmol) conduisent, après chromatographie sur colonne de silice (éluant CH₂Cl₂/MeOH 99/1), au dérivé attendu (170mg, 0.361mmol, 72%) sous forme d'un solide beige.

MS (ES (+)) : m/z [M+H]⁺ = 471

¹H NMR (400 MHz, CDCl₃) : δ (ppm) = 1.20 (s, 9H, C(CH₃)₃), 2.80 (dd, 1H, N-CH₂-CH-N), 2.93 (s, 6H, N(CH₃)₂), 3.09 (m, 2H, CH-CH₂-NHBoc, N-CH₂-CH-N), 3.51 (m, 1H, CH-CH₂-NHBoc), 3.74 (d, 1H, N-CH₂-CH-N), 4.33 (m, 1H, CH-CH₂-NHBoc), 4.69 (AB, 2H, CH₂-Ph), 4.90 (broad, 1H, NH), 7.12-7.18 (m, 5H, Ph), 7.72 (s, 1H, H pyrazole).

### StadeB

### Sel de pyridinium du trans [[4,5,6,8-tétrahydro-2-diméthylcarbamoyle-6-oxo-5-(sulfooxy)-4,7-méthano-7H-pyrazolo[3,4-e] [1,3] diazépin-8-yl]méthyl]-carbamate de 1,1-diméthyléthyle

En procédant comme indiqué au stade D de l'exemple 1, l'utilisation du dérivé obtenu au stade A (176mg, 0.374mmol), de diméthylformamide (0.5mL), de dichlorométhane (1.6mL) et de palladium 10% sur charbon à 50% en eau (119mg, 0.032mmol) conduisent à l'intermédiaire débenzylé attendu. L'intermédiaire débenzylé, la pyridine (1.1mL) et le complexe pyridine / sulfure de trioxyde (119mg, 0.748mmol) conduisent, après chromatographie sur colonne de silice (éluant gradient CH₂Cl₂/MeOH 100/0 à 80/20 par 5%) au dérivé attendu (167mg, 0.309mmol, 83%) sous forme d'un solide beige.

MS (ES(-)) : m/z [M-H]⁻= 459

¹H NMR (400MHz, MeOH-d₄) : δ (ppm) = 1.52 (s, 9H, C(CH₃)₃), 3.23 (s, 6H, N(CH₃)₂), 3.41-3.53, 3.56-3.65 (m, 4H, N-CH₂-CH-N et CH-CH₂-NHBoc), 4.64 (m, 1H, CH-CH₂-NHBoc), 4.98 (d, 1H, N-CH₂-CH-N), 8.07 (m, 2H, Py), 8.20 (s, 1H, H pyrazole), 8.60 (m, 1H, Py), 8.88 (m, 2H, Py)

### Stade C

### Sel de sodium et de trifluoroacétate de la trans 8-(aminométhyl)-2-diméthylcarbamoyle-4,5,6,8-tétrahydro-5-(sulfooxy)-4,7-méthano-7H-pyrazolo[3,4-e] [1,3] diazépin-6(5H)-one

En procédant comme indiqué au stade E de l'exemple 1, l'utilisation du dérivé obtenu au stade B (167mg, 0.309mmol), de résine DOWEX 50WX8 (20g) et de soude 2N (100mL) conduisent au sel de sodium attendu (139mg, 0.288mmol, 93%).

En procédant comme indiqué au stade F de l'exemple 1, le sel de sodium (139mg, 0.288mmol), le dichlorométhane (4mL), l'acide trifluoroacétique (7.9mL) dans le dichlorométhane (7.9mL) conduisent au dérivé brut qui est repris dans l'eau (∼2mL) puis congelé et lyophilisé pour conduire au dérivé attendu (143mg, 0.288mmol, 100%) sous forme d'un solide beige.

¹H NMR (400 MHz, DMSO-d₆) : δ (ppm) = 3.07 (s, 6H, N(CH₃)₂), 3.23-3.27, 3.37-3.42 (m, 4H, N-CH₂-CH-N et CH-CH₂-NH₃⁺), 4.68 (m, 1H, CH-CH₂-NH₃⁺), 4.85 (d, 1H, N-CH₂-CH-N), 8.11 (broad, 3H, NH₃⁺), 8.19 (s, 1H, H pyrazole)

### Exemple 3 : Sel de sodium et de trifluoroacétate de la trans 8-(aminométhyl)-2-méthylcarbamoyl-4,8-dihydro-5-(sulfooxy)-4,7-méthano-7H-pyrazolo[3,4-e] [1,3] diazépin-6(5H)-one

### Stade A

### Trans [[4,5,6,8-tétrahydro-2-méthylcarbamoyl-6-oxo-5-(phénylméthoxy)-4,7-méthano-7H-pyrazolo[3,4-e] [1,3] diazépin-8-yl]méthyl]-carbamate de 1,1-diméthyléthyle

En procédant comme indiqué au stade C de l'exemple 1, la réaction mettant en jeu le dérivé obtenu au stade B de l'exemple 1 (200mg, 0.501mmol), le dichlorométhane (26mL), la triéthylamine (140µL, 1.00mmol), le diphosgène (91µL, 0.751mmol) et une solution de méthylamine (40wt% aqueux, 0.437mL, 5.01mmol) est répétée 2 fois. Les produits bruts sont regroupés et conduisent, après chromatographie sur colonne de silice (CH₂Cl₂/AcOEt 100/0 à 80/20), au dérivé attendu (170mg, 0.372mmol, 60%).

MS (ES(+) : m/z [M+H]⁺ = 457

¹H NMR (400 MHz, CDCl₃) : δ (ppm) = 1.49 (s, 9H, C(CH₃)₃), 3.02 (d, 3H, NH-CH₃), 3.10 (AB, 1H, N-CH₂-CH-N), 3.34-3.38 (m, 2H, N-CH₂-CH-N et CH-CH₂-NHBoc), 3.8 (broad, 1H, CH-CH₂-NHBoc), 4.00 (d, 1H, N-CH₂-CH-N), 4.56-4.60(m, 1H, CH-CH₂-NHBoc), 4.88-5.06 (AB, 2H, N-O-CH₂-Ph), 5.10 (broad, 1H , NH), 6.95 (broad, 1H, NH), 7.42- 7.75 (m, 5H, Ph), 8.07 (s, 1H, H pyrazole).

### Stade B

### Sel de pyridinium du trans [[4,5,6,8-tétrahydro-2-méthylcarbamoyl-6-oxo-5-(sulfooxy)-4,7-méthano-7H-pyrazolo[3,4-e] [1,3] diazépin -8-yl]méthyl]-carbamate de 1,1-diméthyléthyle

En procédant comme indiqué au stade D de l'exemple 1, l la mise en oeuvre du dérivé obtenu au stade A (160mg, 0.350mmol), de diméthylformamide (0.51mL), de dichlorométhane (1.52mL), de palladium 10% sur charbon à 50% en eau (112mg, 0.052mmol) et une hydrogénation pendant 2.h15 conduisent à l'intermédiaire débenzylé attendu.

La mise en oeuvre de l'intermédiaire débenzylé, de pyridine (1.0mL) et de complexe pyridine / sulfure de trioxyde (111mg, 0.699mmol) conduit, après chromatographie sur colonne de silice (éluant CH₂Cl₂/MeOH 100/0 à 80/20), au dérivé attendu (120mg, 0.224mmol, 64%) sous forme d'un solide beige.

MS (ES(+) : m/z [M+H]⁺ = 447) et (ES(-)) : m/z [M-H]⁻ = 445 ¹H NMR (400 MHz, CDCl₃) : δ (ppm) = 1.48 (s, 9H, C(CH₃)₃), 3.01 (d, 3H, NH-CH₃), 3.25 (broad, 1H, N-CH₂-CH-N), 3.40 (broad, 1H, CH-CH₂-NHBoc), 3.7 (broad, 1H, N-CH₂-CH-N), 3.85 (broad, 1H, CH-CH₂-NHBoc) 4.60 (broad, 1H, N-CH₂-CH-N), 5.03(s, 1H , CH-CH₂-NHBoc), 5.40 (broad, 1H , NH), 7.10 (broad, 1H, NH), 7.87-7.91 (m, 2H, Pyridine), 8.20 (s, 1H, H pyrazole), 8.36 (t, 1H, Pyridine), 8.94 (d, 2H, pyridine).

### Stade C

### Sel de sodium du trans [[4,5,6,8-tétrahydro-2-méthylcarbamoyl-6-oxo-5-(sulfooxy)-4,7-méthano-7H-pyrazolo[3,4-e] [1,3] diazépin-8-yl]méthyl]-carbamate de 1,1-diméthyléthyle

En procédant comme indiqué au stade E de l'exemple 1, la mise en oeuvre du dérivé obtenu au stade B (120mg, 0.228mmol) déposé dans un minimum d'eau, de résine DOWEX 50WX8 (20g) et de soude 2N (70mL) conduit au sel de sodium attendu (100mg, 0.213mmol, 93%) sous forme de lyophilisat blanc.

MS (ES(-)) : m/z [M-H]⁻ = 445

¹H NMR (400 MHz, D₂O) : 1.48 (s, 9H, C(CH₃)₃), 2.85 (s, 3H, NH-CH₃), 3.40-3.70 (m, 4H, N-CH₂-CH-N et CH-CH₂-NHBoc), 4.60 (m, 1H, N-CH₂-CH-N), 5.10 (s, 1H , CH-CH₂-NHBoc), 8.23 (s, 1H, H pyrazole).

### Stade D

### Sel de sodium et de trifluoroacétate de la trans 8-(aminométhyl)-2-méthylcarbamoyl-4,8-dihydro-5-(sulfooxy) -4,7-méthano-7H-pyrazolo[3,4-e] [1,3] diazépin-6(5H)-one

En procédant comme indiqué au stade F de l'exemple 1, la mise en oeuvre du sel de sodium obtenu au stade C ((94mg, 0.2mmol), de dichlorométhane (3mL) et d'acide trifluoroacétique (2mL) conduit au dérivé brut qui est repris dans l'eau (10mL) puis congelé et lyophilisé. On obtient le dérivé attendu (95mg, 0.196mmol, 98%) sous forme d'un solide brun.

MS (ES(-)) : : m/z [M-H]⁻ = 345 et ES(+) : m/z [M+H]⁺ = 447

¹H NMR (400 MHz, DMSO-d₆ + 1 goutte D₂O) : 3.77 (s, 3H, NH-CH₃); 3.22-3.48 (m, 4H, N-CH₂-CH-N et CH-CH₂-NHBoc), 4.66-4.70 (m, 1H, N-CH₂-CH-N), 4.84(s, 1H, CH-CH₂-NHBoc), 8.23 (s, 1H, H pyrazole).

### Exemple 4 : Sel de sodium et de trifluoroacétate de la trans 8-(aminométhyl)-1-(2-amino-éthyl)-4,8-dihydro-5-(sulfooxy)-4,7-méthano-7H-pyrazolo[3,4-e] [1,3] diazépin-6(5H)-one

### Stade A

### Trans-1-(2-tert-butoxycarbonylamino-éthyl)-4,5,6,8-tétrahydro-6-oxo-5-(phénylméthoxy)-4,7-méthano-7H-pyrazolo[3,4-e][1,3]diazépine-8-carboxylate de méthyle

### trans-2-(2-tert-butoxycarbonylamino-éthyl)-4,5,6,8-tétrahydro-6-oxo-5-(phénylméthoxy)-4,7-méthano-7H-pyrazolo[3,4-e][1,3]diazépine-8-carboxylate de méthyle

L'ester trans-4,5,6,8-tétrahydro-6-oxo-5-(phénylméthoxy)-4,7-méthano-7H-pyrazolo[3,4-e][1,3]diazépine-8-carboxylate de méthyle décrit dans la demande WO2004/052891 (Exemple 1, stade K) (1.13g, 3.44mmol) est mis en solution dans du diméthylformamide anhydre (4.0mL) en présence de carbonate de potassium (712mg, 5.16mmol) et de bromure de 2-(boc-amino)-éthyle (770mg, 3.44mmol). Le mélange réactionnel est chauffé à 55°C. Des quantités supplémentaires de K2CO3 (2 x 712mg, 2 x 5.16mmol) et de bromure (2 x 770mg, 2 x 3.44mmol) sont ajoutées au bout de 4h et 14h supplémentaires. La réaction est poursuivie encore 8h à 55°°C. La suspension est refroidie, filtrée et rincée par l'acétate d'éthyle. La phase organique est lavée avec une solution d'acide tartrique 10% puis séchée et concentrée sous pression réduite. Le brut est purifié par chromatographie sur silice (éluant gradient CH₂Cl₂/MeOH 100/0 à 90/10) pour conduire au dérivé substitué en N1 (380mg, 0.81mmol, 23%) ainsi qu'à l'isomère substitué en N2 (475mg, 1.01mmol, 29%).

### Dérivé substitué en N1 :

MS (ES(+)) : m/z [M+H]⁺ = 472

1H NMR (400 MHz, CDCl₃) : δ (ppm) = 1.45 (s, 9H, C(CH₃)₃), 3.24 (d, 1H, N-CH₂-CH-N), 3.42 (dd, 1H, N-CH₂-CH-N), 3.50 (m, 1H, CH₂-CH₂-NHBoc), 3.60 (m, 1H, CH₂-CH₂-NHBoc), 3.86 (s, 3H, CH₃), 3.98 (d, 1H, N-CH₂-CH-N), 4.09 (m, 2H, CH₂-CH₂-NHboc), 4.95 (AB, 2H, CH₂-Ph), 5.19 (broad, 1H, NH), 5.23 (s, 1H, CH-CO₂Me), 7.39-7.44 (m, 6H, H pyrazole + Ph)

Dérivé substitué en N2 :

MS (ES(+)) : m/z [M+H]⁺ = 472

¹H NMR (400 MHz, CDCl₃) : δ (ppm) = 1.45 (s, 9H, C(CH₃)₃), 3.48-3.53 (m, 4H, N-CH₂-CH-N, CH₂-CH₂-NHBoc), 3.85 (s, 3H, CH₃), 3.97 (d, 1H, N-CH₂-CH-N), 4.18 (m, 2H, CH₂CH₂-NHboc), 4.95 (AB, 2H, CH₂-Ph), 5.29 (s, 1H, CH-CO₂Me), 7.25 (s, 1H, H pyrazole), 7.38-7.43 (massif, 5H, Ph)

### Stade B

### Trans-1-(2-tert-butoxycarbonylamino-éthyl)-8-(hydroxyméthyl)-4,5,6,8-tétrahydro-5-(phénylméthoxy)-4,7-méthano-7H-pyrazolo[3,4-e][1,3]diazépin-6(5H)-one

En procédant comme indiqué au stade A de l'exemple 1, la mise en oeuvre de l'ester substitué en N1 obtenu au stade A (475mg, 1.0mmol de NaBH₄ (76mg+76mg, 2.0mmol+2.0mmol), de tétrahydrofurane (12.5mL) et de méthanol (12.5mL) à 0°C conduit, après chromatographie sur colonne de silice (éluant gradient CH₂Cl₂/MeOH 100/0 à 90/10) au dérivé attendu (321mg, 0.72mmol, 72%).

¹H NMR (400 MHz, CDCl₃) : δ (ppm) = 1.42 (s, 9H, C(CH₃)₃)), 3.26-3.32 (m, 3H, N-CH₂-CH-N, CH₂-CH₂-NHBoc), 3.50 (m, 2H, N-CH₂-CH-N, CH₂-CH₂-NHboc), 3.95 (d, 1H, N-CH₂-CH-N), 4.06 (m, 3H, CH₂-CH₂-NHBoc, CH-CH₂-OH), 4.62 (m, 1H, CH-CH₂-OH), 4.95 (AB, 2H, CH₂-Ph), 5.28 (broad, 1H, NH), 7.36-7.44 (m, 6H, Ph + H pyrazole).

### Stage C

### Trans [[1-(2-tert-butoxycarbonylamino-éthyl)-4,5,6,8-tétrahydro-6-oxo-5-(phénylméthoxy)-4,7-méthano-7H-pyrazolo[3,4-e][1,3]diazépin-8-yl]méthyl]-carbamate de 1,1-diméthyle

En procédant comme indiqué au stade B de l'exemple 1, la mise en oeuvre de l'alcool obtenu au stade B (320mg, 0.72mmol) dans le dichlorométhane (20mL), de chlorure de méthanesulfonyle (83µL+55µL, 1.08mmol+0.72mmol) et de triéthylamine (151µL+100µL, 1.08mmol+0.72mmol) conduit, après purification par chromatographie sur colonne de silice (éluant gradient CH₂Cl₂/MeOH 100/0 à 90/10) au dérivé mésylé attendu (229mg, 0.44mmol, 61%).

¹H RMN (400 MHz, CDCl₃) : δ (ppm) = 1.46 (s, 9H, C(CH₃)₃), 3.17 (s, 3H, SO₂Me), 3.23 (d, 1H, N-CH₂-CH-N), 3.37 (dd, 1H, N-CH₂-CH-N), 3.54 (m, 2H, CH₂-CH₂-NHBoc), 3.97 (d, 1H, N-CH₂-CH-N), 4.07 (m, 2H, CH₂-CH₂-NHBoc), 4.62 (m, 2H, CH₂-OMs), 4.87 (m, 1H, CH-CH₂-OMs), 4.95 (AB, 2H, CH₂-Ph), 5.06 (broad, 1H, NH), 7.38-7.45 (m, 6H, Ph, H pyrazole).

L'intermédiaire mésylé (300mg, 0.575mmol) dans le diméthylformamide (4mL) et NaN₃ (75mg+75mg, 1.15mmol+1.15mmol) conduisent à l'azide attendu.

¹H RMN (400 MHz, CDCl₃) : δ (ppm) = 1.43 (s, 9H, C(CH₃)₃), 3.24 (d, 1H, N-CH₂-CH-N), 3.31 (dd, 1H, N-CH₂-CH-N), 3.49 (m, 2H, CH₂-CH₂-NHBoc), 3.75 (m, 2H, CH₂-N₃), 3.94 (d, 1H, N-CH₂-CH-N), 3.99 (m, 2H, CH₂-CH₂-NHBoc), 4.68 (dd, 1H, CH-CH₂-N₃), 4.91 (AB, 2H, CH₂-Ph), 5.17 (broad, 1H, NH), 7.33-7.41 (m, 6H, Ph, H pyrazole).

La triméthylphosphine (solution 1M dans le tétrahydrofurane, 748µL, 0.75mmol) est ajoutée 0°C à une solution de l'azide obtenu ci-dessus(320mg, 0.575mmol) dans le tétrahydrofurane (2.5mL) et le toluène (2.5mL). Cette solution est agitée 2h à température ambiante, puis refroidie à 0°C et une solution de BOC-ON (212mg, 0.86mmol) dans le tétrahydrofurane (2mL) est ajoutée. La solution est agitée 1h à température ambiante, puis hydrolysée par ajout d'une solution saturée de NaHCO₃ puis extraite avec l'acétate d'éthyle. Les phases organiques rassemblées sont séchées puis concentrées. Le résidu est purifié par chromatographie sur colonne de silice (éluant gradient cyclohexane/acétate d'éthyle 60/40 à 30/70) pour fournir le dérivé attendu (220mg, 0.41mmol, 70%).

MS (ES(+)) : m/z [M+H]⁺ = 543

¹H RMN (400 MHz, CDCl₃) : δ (ppm) = 1.44 (s, 9H, C(CH₃)₃), 1.45 (s, 9H, C(CH₃)₃), 3.13 (d, 1H, N-CH₂-CH-N), 3.25 (m, 2H, N-CH₂-CH-N, CH-CH₂-NHBoc), 3.56 (m, 2H, CH₂-CH₂-NHBoc), 3.75 (m, 1H, CH-CH₂-NHBoc), 3.95 (d, 1H, N-CH₂-CH-N), 4.11 (m, 2H, CH₂-CH₂-NHBoc), 4.55 (dd, 1H, CH-CH₂-NHBoc), 4.92 (AB, 2H, CH₂-Ph), 5.29 (broad, 2H, NH), 7.35-7.43 (m, 6H, Ph, H pyrazole).

### Stade D

### Sel de sodium et de trifluoroacétate de la trans 8-(aminométhyl)-1-(2-amino-éthyl)-4,8-dihydro-5-(sulfooxy)-4,7-méthano-7H-pyrazolo[3,4-e][1,3]diazépin-6(5H)-one

En procédant comme indiqué au stade D de l'exemple 1, la mise en oeuvre du composé obtenu au stade C (210mg, 0.387mmol) dans le diméthylformamide (1mL) et de dichlorométhane (3mL), Pd/C (50% H₂O, 75mg+40mg) conduisent au dérivé débenzylé attendu.

La mise en oeuvre de l'intermédiaire débenzylé, du complexe pyridine/sulfure de trioxyde (123mg, 0.775mmol) et de la pyridine (2mL) conduit après purification par chromatographie sur colonne de silice (éluant gradient CH₂Cl₂/MeOH 100/0 à 80/20) au sel de pyridinium attendu (230mg, 0.387mmol, 100%). En procédant comme indiqué au stade C de l'exemple 2, la mise en oeuvre de sel de pyridinium obtenu ci-dessus (230mg, 0.387mmol), d'une solution de soude 2N (50mL) et de résine DOWEX 50WX8 (18g) conduit au sel de sodium attendu (121mg, 0.22mmol, 56%) sous forme d'une poudre blanche.

MS (ES(-)) : m/z [M-H]⁻= 531

¹H RMN (400 MHz, DMSO-d₆): δ (ppm) = 1.37 (s, 9H, C(CH₃)₃), 1.41 (s, 9H, C(CH₃)₃), 3.20-3.33 (m, 5H, N-CH₂-CH-N, CH-CH₂-NHBoc, CH₂-CH₂-NHBoc), 3.43 (m, 1H, CH-CH₂-NHBoc), 3.99 (m, 2H, CH₂-CH₂-NHBoc), 4.44 (dd, 1H, CH-CH₂-NHBoc), 4.65 (d, 1H, N-CH₂-CH-N), 6.92 (broad, 1H, NH), 7.11 (broad, 1H, NH), 7.43 (s, 1H, H pyrazole).

La mise en oeuvre du sel de sodium (55mg, 0.099mmol) dans le dichlorométhane (1.5mL) et d'un mélange d'acide trifluoroacétique (3mL) et de dichlorométhane (3mL) conduit au sel de sodium et de trifluoroacétate attendu (47mg, 0.081mmol, 70%) sous la forme d'une poudre crème.

¹H RMN (400 MHz, DMSO-d₆): δ (ppm) = 3.26-3.42 (m, 6H, N-CH₂-CH-N, CH-CH₂-NH₃⁺, CH₂-CH₂-NH₃⁺), 4.23 (m, 2H, CH₂-CH₂-NH₃⁺), 4.78 (m, 2H, CH-CH₂-NH₃⁺, N-CH₂-CH-N), 7.60 (s, 1H, H pyrazole), 8.02 (broad, 3H, NH₃⁺), 8.19 (broad, 3H, NH₃⁺).

### Exemple 5 : Sel de sodium et de trifluoroacétate de la trans 8-(aminométhyl)-2-(2-amino-éthyl)-4,8-dihydro-5-(sulfooxy)-4,7-méthano-7H-pyrazolo[3,4-e][1,3]diazépin-6(5H)-one

### Stade A

### Trans-2-(2-tert-butoxycarbonylamino-éthyl)-8-(hydroxyméthyl)-4,8-dihydro-5-(phénylméthoxy)-4,7-méthano-7H-pyrazolo[3,4-e][1,3]diazépin-6(5H)-one

En procédant comme indiqué au stade A de l'exemple 1, la mise en oeuvre de l'ester substitué en N2 obtenu au stade A de l'exemple 4 (623mg, 1.32mmol), de NaBH₄ (300mg, 7.92mmol), de tétrahydrofurane (13mL) et de méthanol (13mL) à 0°C conduit, après chromatographie sur colonne de silice (éluant gradient CH₂Cl₂/MeOH 98/2 à 90/10) au dérivé attendu (250mg, 0.58mmol, 43%).

MS (ES(+)) : m/z [M+H]⁺ = 444

¹H NMR (400 MHz, CDCl₃) : δ (ppm) = 1.40 (s, 9H, C(CH₃)₃), 3.24 (d, 1H, N-CH₂-CH-N), 3.31 (dd, 1H, N-CH₂-CH-N), 3.35 (m, 1H, CH₂-CH₂-NHBoc), 3.48 (m, 1H, CH₂-CH₂-NHBoc), 3.89-4.11 (m, 5H, CH₂-CH₂-NHBoc, N-CH₂-CH-N, CH-CH₂-OH), 4.61 (dd, 1H, N-CH-CH₂-N), 4.92 (AB, 2H, CH₂-Ph), 5.18 (broad, 1H, NH), 7.21 (s, 1H, H pyrazole), 7.33-7.42 (m, 5H, Ph).

### Stade B

### Trans [[2-(2-tert-butoxycarbonylamino-éthyl)-4,5,6,8-tétrahydro-6-oxo-5-(phénylméthoxy)-4,7-méthano-7H-pyrazolo[3,4-e][1,3]diazépin-8-yl]méthyl]-carbamate de 1,1-diméthyle

En procédant comme indiqué au stade C de l'exemple 4, la mise en oeuvre de l'alcool obtenu au stade A (450mg, 1.05mmol) dans le dichlorométhane (30mL), de chlorure de méthanesulfonyl (131µL, 1.68mmol) et de triéthylamine (237µL, 1.68mmol) conduisent au dérivé mésylé attendu (532mg, 1.02mmol, 97%).

MS (ES(+)) : m/z [M+H]⁺ = 522

¹H NMR (400 MHz, CDCl₃) : δ (ppm) = 1.45 (s, 9H, C(CH₃)₃), 3.15 (s, 3H, SO₂CH₃), 3.20 (d, 1H, N-CH₂-CH-N), 3.40 (dd, 1H, N-CH₂-CH-N), 3.50 (m, 2H, CH₂-CH₂-NHboc), 3.98 (d, 1H, N-CH₂-CH-N), 4.13 (m, 2H, CH₂-CH₂-NHBoc), 4.61 (m, 2H, CH₂-OMs), 4.88 (m, 1H, CH-CH₂-OMs), 4.95 (AB, 2H, CH₂-Ph), 7.24 (s, 1H, H pyrazole), 7.37-7.45 (m, 5H, Ph).

La mise en oeuvre de l'intermédiaire mésylé (532mg, 1.05mmol) dans le diméthylformamide (7.5mL) et de NaN₃ (615mg, 9.45mmol) conduit à l'azide attendu (566 mg, 1.05mmol).

¹H NMR (400 MHz, CDCl₃) : δ (ppm) = 1.41 (s, 9H, C(CH₃)₃)), 3.20 (d, 1H, N-CH₂-CH-N), 3.35 (dd, 1H, N-CH₂-CH-N), 3.44 (m, 2H, CH₂-CH₂-NHBoc), 3.65 (m, 2H, CH₂-N₃), 3.95 (d, 1H, N-CH₂-CH-N),

4.09 (m, 2H, CH₂-CH₂-NHBoc), 4.71 (dd, 1H, CH-CH₂-N₃), 4.92 (AB, 2H, CH₂-Ph), 4.98 (broad, 1H, NH), 7.21 (s, 1H, H pyrazole), 7.33-7.41 (m, 5H, Ph).

La mise en oeuvre de l'azide ci-dessus (565mg, 1.05mmol), de triméthylphosphine (solution 1M dans le tétrahydrofurane, 1.36mL, 1.36mmol), de BOC-ON (388mg, 1.58mmol), de tétrahydrofurane (5.5mL) et de toluène (3mL) conduit au produit attendu (205mg, 0.38mmol, 36%).

MS (ES(+)) : m/z [M+H]⁺ = 543

¹H NMR (400 MHz, CDCl₃) : δ ppm) = 1.45 (s, 9H, C(CH₃)₃), 1.46 (s, 9H, C(CH₃)₃), 3.10 (d, 1H, N-CH₂-CH-N), 3.29 (dd, 1H, N-CH₂-CH-N), 3.37 (m, 1H, CH-CH₂-NHBoc), 3.49 (m, 2H, CH₂-CH₂-NHBoc), 3.69 (m, 1H, CH-CH₂-NHBoc), 3.94 (d, 1H, N-CH₂-CH-N), 4.10 (m, 2H, CH₂-CH₂-NHBoc), 4.58 (dd, 1H, CH-CH₂-NHBoc), 4.91 (broad, 1H, NH), 4.92 (AB, 2H, CH₂-Ph), 5.13 (broad, 1H, NH), 7.20 (s, 1H, H pyrazole), 7.37-7.44 (m, 5H, Ph).

### Stade C

### Sel de sodium et de trifluoroacétate de la trans 8-(aminométhyl)-2-(2-amino-éthyl)-4,8-dihydro-5-(sulfooxy)-4,7-méthano-7H-pyrazolo[3,4-e][1,3]diazépin-6(5H)-one

En procédant comme indiqué au stade D de l'exemple 1, la mise en oeuvre du composé obtenu au stade B (85mg, 0.157mmol) dans un mélange diméthylformamide/dichlorométhane (1/3, 2mL) et de Pd/C (50% H₂O, 30mg) conduit au dérivé débenzylé attendu.

La mise en oeuvre de l'intermédiaire débenzylé obtenu, de complexe pyridine/sulfure de trioxyde (50mg, 0.314mmol) et de pyridine (0.75mL) conduit, après purification par chromatographie sur colonne de silice (éluant gradient CH₂Cl₂/MeOH 98/2 à 80/20), au sel de pyridinium attendu (85mg, 0.139mmol, 86%).

En procédant comme indiqué au stade C de l'exemple 2, la mise en oeuvre de sel de pyridinium (85mg, 0.139mmol), d'une solution de soude 2N (42mL) et de résine DOWEX 50WX8 (8.5g) conduit au sel de sodium attendu (75mg, 0.135mmol, 86%), sous forme d'une poudre crème.

MS (ES(-)) : m/z [M⁻] = 531

¹H NMR (400 MHz, DMSO-d₆) : δ (ppm) = 1.37 (s, 9H, C(CH₃)₃), 1.40 (s, 9H, C(CH₃)₃)), 3.17-3.32 (m, 5H, N-CH₂-CH-N, CH-CH₂-NHBoc, CH₂-CH₂-NHBoc), 3.60 (m, 1H, CH-CH₂-NHBoc), 4.04 (m, 2H, CH-CH₂-NHBoc), 4.31 (dd, 1H, CH-CH₂-NHBoc), 4.65 (s, 1H, N-CH₂-CH-N), 6.94 (broad, 2H, NH), 7.65 (s, 1H, H pyrazole).

La mise en oeuvre du sel de sodium (75mg, 0.135mmol) dans le dichlorométhane (2mL) et d'un mélange d'acide trifluoroacétique (4mL) et de dichlorométhane (4mL) conduit au sel de sodium et de trifluoroacétate (35mg, 0.059mmol, 44%) sous la forme d'une poudre crème.

¹H NMR (400 MHz, DMSO-d₆) : δ (ppm) = 3.20-3.41 (m, 6H, N-CH₂-CH-N, CH-CH₂-NNH₃⁺, CH₂-CH₂-NH₃⁺), 4.30 (m, 2H, CH₂-CH₂-NH₃⁺), 4.63 (dd, 1H, CH-CH₂-NH₃⁺), 4.77 (d, 1H, N-CH₂-CH-N), 7.85 (s, 1H, H pyrazole), 8.04 (broad, 3H, NH₃⁺), 8.17 (broad, 3H, NH₃⁺).

### Exemple 6 : Sel de sodium et de trifluoroacétate de la trans 8-(aminométhyl)-2-(2-pyridinyl)-4,8-dihydro-5-(sulfooxy)-4,7-méthano-7H-pyrazolo[3,4-e][1,3]diazépin-6(5H)-one

### Stade A

### Trans [[4,5,6,8-tétrahydro-2-(2-pyridinyl)-6-oxo-5-(phénylméthoxy)-4,7-méthano-7H-pyrazolo[3,4-e][1,3]diazépin-8-yl]méthyl]-carbamate de 1,1-diméthyléthyle

Le dérivé obtenu au stade B de l'exemple 1 (0.500g, 1.248mmol), la 2-bromopyridine (217mg, 1,373mmol), la L-proline (32mg, 0.275mmol), l'iodure de cuivre (24mg, 0.125mmol) et le carbonate de potassium (345mg, 2.497mmol) sont mis en suspension dans du diméthylsulfoxyde anhydre (1.875mL). La réaction est ensuite poursuivie sous azote, en tube scellé à 100°C pendant 48h. Le mélange réactionnel est ensuite traité à l'eau, puis extrait au dichlorométhane. La phase organique est ensuite séchée puis concentrée. Le brut ainsi obtenu est alors purifié par chromatographie sur silice (éluant CH₂Cl₂/MeOH 98/2 puis 95/5) pour donner le produit attendu (91mg, 0.189mmol, 15%).

MS (ES(+)) : m/z [M+H]⁺ = 477

¹H RMN (400MHz, MeOD-d₄) : δ (ppm) = 1.51 (s, 9H, tBu), 3.37-3.39 (m, 4H, N-CH₂-CH-N, N-CH-CH₂-NHBOC), 4.44 (d, 1H, N-CH-CH₂-NHBOC), 4.65 (dd, 1H, N-CH₂-CH-N), 4.98 (AB, 2H, CH₂Ph), 7.25-7.53 (m, 6H, Ph, pyridine), 7.90 (m, 2H, pyridine), 8.42 (d, 1H, pyridine), 8.51 (s, 1H, pyrazole).

### Stade B

### Sel de sodium du trans [[4,5,6,8-tétrahydro-2-(2-pyridinyl)-6-oxo-5-(sulfooxy)-4,7-méthano-7Hpyrazolo[3,4-e][1,3]diazépin-8-yl]méthyl]-carbamate de 1,1-diméthyléthyle

En procédant comme indiqué au stade D de l'exemple 1, la mise en oeuvre du dérivé obtenu au stade A (90mg, 0.189mmol), de mélange diméthylformamide / dichlorométhane 1/3 (2.0mL) et de palladium 10% sur charbon à 50% en eau (36mg) conduit après 3 jours sous hydrogène à l'intermédiaire débenzylé attendu.

La mise en oeuvre de l'intermédiaire débenzylé, de pyridine (0.73mL) et de complexe pyridine / sulfure de trioxyde (60mg, 0.378mmol) conduit, après chromatographie sur colonne de silice (éluant CH₂Cl₂/MeOH 90/10), au dérivé attendu (63mg).

Le brut est ensuite repris dans de la pyridine (0.73mL), sous azote, en présence de complexe SO₃/pyridine (60mg, 0.378mmol). Le mélange réactionnel est ensuite agité à température ambiante jusqu'à conversion complète en HPLC (72h). Après traitement par ajout d'H₂O, le mélange est filtré puis évaporé à sec. Le brut ainsi obtenu est purifié par chromatographie sur silice (éluant CH₂Cl₂/MeOH 90/10). Le produit est ainsi obtenu pur (63mg).

Une suspension de 8.5g de résine DOWEX 50WX8 dans une solution de soude 2N (43mL) est agitée pendant 1h, puis versée sur une colonne à chromatographie. La colonne est conditionnée à l'eau déminéralisée jusqu'à pH neutre. Le dérivé obtenu (63mg) est dissout dans un minimum de méthanol et d'eau, déposé sur la colonne, puis élué avec H₂O. Les fractions contenant le substrat sont réunies, congelées et lyophilisées pour conduire au sel de sodium attendu (55mg, 0.112mmol, 60%) sous forme d'une poudre jaune.

MS (ES (-)) : m/z [M-H]⁻ = 465

¹H RMN (400MHz, MeOD-d₄) : δ (ppm) = 1.53 (s, 9H, ^{t}Bu), 1.54 (m, 4H, N-CH₂-CH-N, N-CH-CH₂-NHBoc), 4.58 (dd, 2H, N-CH-CH₂-NHBoc), 5.02 (d, 1H, N-CH₂-CH-N), 7.34 (m, 1H, pyridine), 7.97 (m, 2H, pyridine), 8.47 (d, 1H, pyridine), 8.65 (s, 1H, H pyrazole).

### Stade C

### Sel de sodium et de trifluoroacétate de la trans 8-(aminométhyl)-2-(2-pyridinyl)-4,8-dihydro-5-(sulfooxy)-4,7-méthano-7H-pyrazolo[3,4-e][1,3]diazépin-6(5H)-one

En procédant comme indiqué au stade F de l'exemple 1, la mise en oeuvre du sel de sodium obtenu au stade B (55mg, 0.112mmol), de dichlorométhane anhydre (1.92mL), et de mélange acide trifluoroacétique/dichlorométhane 1/1 (7.68mL) conduit à un dérivé brut qui est repris dans l'eau puis lavé à l'éther diethylique. L'insoluble est filtré et séché sous pression réduite pour donner le produit attendu (20mg, 0.04mmol, 35%) sous forme de poudre beige.

MS (ES (+)) : m/z [M+H]⁺ = 367

¹H RMN (400MHz, DMSO-d₆) : δ (ppm) = 3.30-3.49 (2 ABX, 4H, N-CH₂-CH-N, N-CH-CH₂-NH₃⁺), 4.75 (dd, 2H, N-CH-CH₂-NH₃⁺), 4.92 (m, 1H, N-CH₂-CH-N), 7.35 (m, 1H, pyridine), 7.83 (d, 1H, pyridine), 7.95 (m, 1H, pyridine), 8.49 (m, 1H, pyridine), 8.61 (s, 1H, H pyrazole).

### Exemple 7 : Sel de sodium et de trifluoroacétate du trans-8-(aminométhyl)-5,6-dihydro-6-oxo-5-(sulfooxy)-4H-4,7-méthanopyrazolo[3,4-e][1,3]diazépin-2(8H)-acétamide

### Stade A

### Trans-5,6-dihydro-8-(tert-butoxycarbonylaminométhyl)-6-oxo-5-(phénylméthoxy)-4H-4,7-méthanopyrazolo[3,4-e][1,3]diazépine-2(8H)acétamide

Le dérivé obtenu au stade B de l'Exemple 1 (1g, 2.5mmol) est mis en solution dans du diméthylformamide anhydre (2.5mL). Du 2-bromoacetamide (829mg, 6mmol) et du carbonate de potassium (692mg, 5mmol) sont ajoutés. La réaction est agitée, sous azote, en tube scellé à 75°C. Du 2-bromoacetamide (1 eq.) et du K₂CO₃ (1 eq.) sont ajoutés après une nuit, et la réaction est poursuivie pendant 4 jours (∼60% de conversion). Le mélange réactionnel est traité avec H₂O puis extrait au dichlorométhane. Les phases organiques combinées sont ensuite séchées sur sulfate de sodium, filtrées puis concentrées. Le brut ainsi obtenu est purifié par chromatographie sur silice (éluant gradient CH₂Cl₂/MeOH 100/0 à 95/5) pour donner le produit attendu (188mg, 0.41mmol, 16%) sous forme de mélange d'isomères N1/N2 dans un rapport d'environ 1/2.

Isomère N2 :

MS (ES (+)) : m/z [M+H]⁺ = 457

¹H RMN (400MHz, DMSO-*d₆*) : δ (ppm) = 1.39 (s, 9H, C(CH₃)₃), 3.12-3.33 (m, 4H, N-CH₂-CH-N, N-CH-CH₂-NHBoc), 4.31 (m, 1H, N-CH-CH₂-NHBoc), 4.40 (m, 1H, N-CH₂-CH-N), 4.66 (s, 2H, CH₂CONH₂), 4.89 (s, 2H, CH₂Bn), 6.99 (m, 1H, NHBOC), 7.58-7.62 (m, 5H, Ph), 7.66 (s, 1H, pyrazole).

### Stade B

### Sel de sodium du trans-5,6-dihydro-8-(tert-butoxycarbonylaminométhyl)-6-oxo-5-(sulfooxy)-4H-4,7-méthanopyrazolo[3,4-e][1,3]diazépine-2(8H)acétamide

En procédant comme au stade B de l'Exemple 7, le composé obtenu au stade A (188mg, 0.411mmol) est hydrogéné, puis sulfaté en présence de complexe SO3/pyridine (131mg, 0.823mmol) dans la pyridine (1.58mL), sous azote, à température ambiante pendant 4 jours. Le produit brut obtenu est purifié par chromatographie sur silice (éluant gradient CH₂Cl₂/MeOH/NH₄OH 80/20/1)pour conduire au produit attendu (23mg, 0.044mmol, 11%) sous la forme d'un mélange d'isomères N1/N2 dans un rapport d'environ 1/2.

Isomère N2 :

MS (ES (+)) : m/z [M+H]⁺ = 447

¹H RMN (400MHz, DMSO-*d₆*) : δ (ppm) = 1.41 (s, 9H, C(CH₃)₃), 3.24-3.32 (m, 4H, N-CH₂-CH-N, N-CH-CH₂-NHBoc), 4.36 (m, 1H, N-CH-CH₂-NHBoc), 4.67 (m, 1H, N-CH₂-CH-N), 4.69 (s, 2H, CH₂CONH₂), 7.02 (m, 1H, NHBOC), 7.40 (s, 2H, NH₂), 7.65 (s, 1H, pyrazole).

L'échange d'ion est réalisé sur résine DOWEX 50WX8 (4g) comme indiqué au stade B de l'Exemple7 pour donner après lyophilisation le sel de sodium attendu (17mg, 0.126mmol, 35%) sous forme d'une poudre beige.

MS (ES (-)) : m/z [M-H]⁻ = 445

### Stade C

### Sel de sodium et de trifluoroacétate du trans-8-(aminométhyl)-5,6-dihydro-6-oxo-5-(sulfooxy)-4H-4,7-méthanopyrazolo[3,4-e] [1,3]diazépine-2(8H)acétamide

Le composé obtenu au stade B (17mg, 0.036mmol) est placé en suspension dans du dichlorométhane anhydre (0.07mL), sous azote. De l'acide trifluoroacétique (0.027mL) est ensuite ajouté goutte à goutte puis la réaction est poursuivie à température ambiante pendant trois heures. Après évaporation à sec, le produit est ensuite repris dans l'eau, gelé puis lyophilisé pour conduire au produit attendu (17mg, 0.035mmol, 98%) sous forme de solide beige, en mélange d'isomères N1/N2 dans un rapport d'environ 1/2.

Isomère N2

MS (ES (-)) : m/z [M-H]⁻ = 345

¹H RMN (400MHz, MeOD-*d₄*) : δ (ppm) = 3.31-3.36 (m, 4H, N-CH₂-CH-N, N-CH-CH₂-NH₃⁺), 4.60 (m, 1H, N-CH-CH₂-NH₃⁺), 4.71 (m, 1H, N-CH₂-CH-N), 4.74 (s, 2H, CH₂CONH₂), 7.25 (broad s, 1H, NH), 7.45 (broad s, 1H, NH), 7.73 (s, 1H, pyrazole), 8.04 (sl, 1H ,NH₃⁺).

### Composition pharmaceutique

On a préparé une composition pour injection renfermant:
- Composé de l'exemple 1: 500 mg
- Excipient aqueux stérile: q.s.p. 5 cm³

### ETUDE PHARMACOLOGIQUE DES COMPOSES DE L'INVENTION

### Activité in vitro, méthode des dilutions en milieu liquide :

On prépare une série de tubes dans lesquels on répartit la même quantité de milieu nutritif stérile, on distribue dans chaque tube des quantités croissantes du produit à étudier puis chaque tube est ensemencé avec une souche bactérienne. Après incubation de 24 heures à l'étuve à 37°C, l'inhibition de la croissance est appréciée par transillumination, ce qui permet de déterminer les concentrations minimales inhibitrices (C.M.I.) exprimées en µg/ml.

On effectue ainsi des tests avec les produits des exemples 1 à 7 en comparaison avec les produits des exemples 7, 9, 11 et 45 de la demande WO 04/052891. Les produits de la présente demande se révèlent très actifs sur Pseudomonas aeruginosa, ce qui n'est absolument pas le cas des produits de comparaison. La différence d'activité sur *Pseudomonas aeruginosa* entre les produits de l'invention et les produits les plus proches de l'art antérieur se situe selon les produits dans une fourchette de 16 à plus de 500.

**Activité sur Pseudomonas aeruginosa (1771 Souche type sauvage)**

| Molécule | MIC (µg/mL) 24h (P, aerug, 1771) |
|---|---|
| Ex 1 | 0.25 |
| Ex 2 | 2 |
| Ex 3 | 2 |
| Ex 4 | 0.25 |
| Ex 5 | 0.25 |
| Ex 6 | 8 |
| Ex 7 | 0.5 |
| Ex 7 Demande de brevet WO 04/052891 | >128 |
| Ex 9 Demande de brevet WO 04/052891 | >128 |
| Ex 11 Demande de brevet WO 04/052891 | >128 |
| Ex 45 Demande de brevet WO 04/052891 | >128 |
| IMP | 1 |
| CAZ | 1 |

IMP= Imipénem et CAZ= Ceftazidime. (Résultats donnés à titre indicatifs).

## Revendications

1. Les composés de formule générale (I), sous leurs formes énantiomères ou diastéroisomères ou isomères cis ou trans possibles, ou de mélanges: 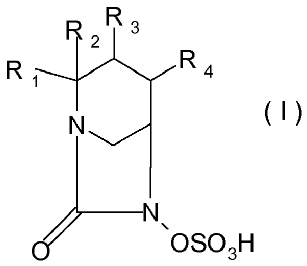 dans laquelle:
R₁ représente un radical (CH₂)ₙ-NH₂ n étant égal à 1 ou 2;
R₂ représente un atome d'hydrogène;
R₃ et R₄ forment ensemble un hétérocycle azoté à caractère aromatique à 5 sommets renfermant 1, 2 ou 3 atomes d'azote, substitué sur cet atome d'azote ou sur l'un de ces atomes d'azote par un groupe (CH₂)ₘ-(C(O))ₚ-R₅, m étant égal à 0, 1, 2 ou 3, p étant égal à 0 ou 1 et R₅ représentant un groupe hydroxy, auquel cas p est égal à 1, ou un groupe amino, alkyl(C₁-C₆) ou dialkyl(C₁-C₆) amino, ou un hétérocycle azoté à caractère aromatique à 5 ou 6 sommets renfermant 1 ou 2 atomes d'azote et, le cas échéant,un atome d'oxygène ou de soufre ;
étant entendu que lorsque le sous-groupe (C(O))ₚ-R₅ forme un groupe carboxy, amino, alkyl(C₁-C₆) ou dialkyl(C₁-C₆) amino, m est différent de 0 ou 1;
sous forme libre et sous forme de zwitterions et de sels avec les bases et les acides minéraux ou organiques pharmaceutiquement acceptables.

2. Les composés de formule générale (I) selon la revendication 1, **caractérisés en ce que** R₃ et R₄ forment ensemble un radical pyrazolyle substitué.

3. Les composés de formule générale (I) selon la revendication 1 ou 2, **caractérisés en ce que** R₁ est un groupement (CH₂)ₙ-NH₂, n étant égal à 1, et l'hétérocycle formé par R₃ et R₄ est substitué par un groupement (CH₂)ₘ-(C(O))ₚ-R₅ tel que défini à la revendication 1.

4. Les composés de formule générale (I) selon la revendication 1, 2 ou 3, **caractérisés en ce que** R₅ représente un groupe amino, alkyl(C₁-C₆) ou dialkyl(C₁-C₆) amino.

5. L'un quelconque des composés de formule générale (I) selon la revendication 1 dont les noms suivent:
- la trans 8-(aminométhyl)-2-carbamoyle-4,8-dihydro-5-(sulfooxy)-4,7-méthano-7H-pyrazolo[3,4-e][1,3]diazépin-6(5H)-one,
- la trans 8-(aminométhyl)-2-diméthylcarbamoyle-4,8-dihydro-5-(sulfooxy)-4,7-méthano-7H-pyrazolo[3,4-e][1,3]diazépin-6(5H)-one,
- la trans 8-(aminométhyl)-2-méthylcarbamoyle-4,8-dihydro-5-(sulfooxy)-4,7-méthano-7H-pyrazolo[3,4-e][1,3]diazépin-6(5H)-one,
- la trans 8-(aminométhyl)-1-(2-aminoéthyl)-4,8-dihydro-5-(sulfooxy)-4,7-méthano-7H-pyrazolo[3,4-e][1,3]diazépin-6(5H)-one,
- la trans 8-(aminométhyl)-2-(2-aminoéthyl)-4,8-dihydro-5-(sulfooxy)-4,7-méthano-7H-pyrazolo[3,4-e][1,3]diazépin-6(5H)-one,
- la trans 8-(aminométhyl)-2-(2-pyridinyl)-4,8-dihydro-5-(sulfooxy)-4,7-méthano-7H-pyrazolo[3,4-e][1,3]diazépin-6(5H)-one,
- le trans 8-(aminométhyl)-5,6-dihydro-6-oxo-5-(sulfooxy)-4H-4,7-méthanopyrazolo[3,4-e][1,3]diazépin-2(8H)-acétamide,
sous forme libre, de zwitterions et de sels avec les bases et les acides minéraux ou organiques pharmarceutiquement acceptables et sous ses formes énantiomères ou diastéroisomères ou isomères cis ou trans possibles, ou de mélanges.

6. Procédé de préparation des composés de formule (I), tel que défini à la revendication 1, **caractérisé en ce que** l'on traite un composé de formule (II) : 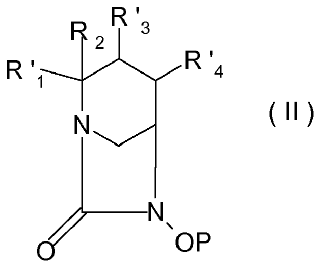 dans laquelle R'₁ représente un radical R₁ dans lequel l'atome d'azote est protégé, R₂ est tel que défini à la revendication 1, R'₃ et R'₄ forment ensemble un hétérocycle azoté à caractère aromatique à 5 sommets renfermant 1, 2 ou 3 atomes d'azote et P représente un groupement protecteur du radical hydroxy, en présence d'une base, par un composé de formule (III) :
X-(CH₂)ₘ-(C(O))ₚ-R'₅ (III)
dans laquelle X représente un atome d'halogène ou un groupement OH pouvant être activé, m et p sont tels que définis à la revendication 1 et R'₅ représente un radical R₅ dans lequel le groupe réactif amino ou carboxy est le cas échéant protégé, pour obtenir un composé de formule (IV): 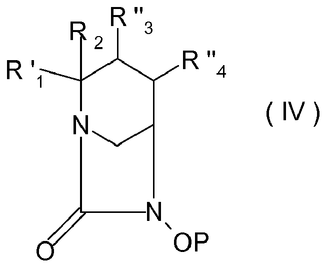 dans laquelle R'₁, R₂ et P sont tels que définis plus haut et R''₃ et R''₄ forment ensemble un hétérocycle azoté à caractère aromatique à 5 sommets tel que défini plus haut pour R₃ et R₄, substitué par un groupement (CH₂)ₘ-(C(O))ₚ-R'₅, m, p et R'₅ étant tels que définis plus haut,
puis déprotège le radical hydroxy et soumet le composé obtenu à une réaction de sulfatation par action de SO₃ complexé, puis, le cas échéant, soumet le composé obtenu à une ou plusieurs des réactions suivantes, dans un ordre approprié :
- déprotection de la ou des fonctions aminées et le cas échéant carboxy présentes,
- salification,
- échange d'ions,
- dédoublement ou séparation de diastéréoisomères.

7. Procédé de préparation des composés de formule (I) dans lesquels m est égal à 0, p est égal à 1 et R₅ représente R''₅, R''₅ représentant un groupe amino, alkyl(C₁-C₆) ou dialkyl (C₁-C₆) amino, **caractérisé en ce que** l'on traite un composé de formule (II) telle que définie à la revendication 6, en présence d'une base, par le diphosgène, puis par une amine de formule
H-R''₅
dans laquelle R''₅ est tel que défini ci-dessus, pour obtenir un composé de formule (IV') : 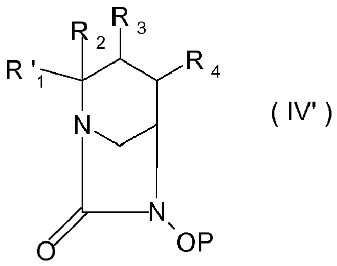 dans laquelle R'₁, R₂ et P sont tels que définis à la revendication 6 et R₃ et R₄ forment ensemble un hétérocycle azoté à caractère aromatique à 5 sommets tel que défini à la revendication 6, substitué par un groupement -C(O)-R''₅, R''₅ étant tel que défini ci-dessus, puis poursuit la synthèse comme décrit à la revendication 6.

8. A titre de médicaments, les composés tels que définis à l'une quelconque des revendications 1 à 4, ainsi que leurs sels avec les acides et les bases pharmaceutiquement acceptables.

9. A titre de médicaments, les composés tels que définis à la revendication 5.

10. Compositions pharmaceutiques contenant, à titre de principe actif, au moins un médicament selon l'une des revendications 8 ou 9.
